# EUROPEAN PATENT SPECIFICATION

(11) **EP 0 848 821 B1**
(45) Date of publication and mention of the grant of the patent: **27.10.1999**
(21) Application number: 95923250.5
(22) Date of filing: 07.06.1995
(51) Int. Cl.: G01N 33/576

(54) **IgG/IgM RATIO FOR DETERMINING EFFECTIVENESS OF INTERFERON THERAPY IN INDIVIDUALS WITH HCV INFECTIONS**
IGG/IGM-VERHÄLTNIS ZUR BESTIMMUNG DER WIRKSAMKEIT VON INTERFERON THERAPIE IN EINZELNER MIT HCV-INFEKTIONEN
RAPPORT IgG/IgM POUR DETERMINER L'EFFICACITE DE LA THERAPIE PAR INTERFERONS CHEZ DES INDIVIDUS ATTEINTS D'INFECTIONS VHC

(43) Date of publication of application: 24.06.1998
(73) Proprietor: ABBOTT LABORATORIES, Abbott Park, Illinois 60064-3500 (US)
(72) Inventor: BRAUN, Hans-Bertram, D-65203 Wiesbaden (DE); MICHEL, Gerd, D-69121 Heidelberg (DE)
(74) Representative: Modiano, Guido, Dr.-Ing.
(86) International application number: EP9502193
(87) International publication number: WO9641195

(56) References cited:
- WO-A-93/20445
- WO-A-94/04922
- BIOLOGICAL ABSTRACTS, vol. 74, no. 4, 1982 Philadelphia, PA, US; abstract no. 24584, H. ZHUANG ET AL. 'Serum immunoglobulin levels in acute A, B and non-A, non-B hepatitis' page 2553; & GASTROENTEROLOGY, vol. 82, no. 3, 1982 pages 549-553,
- BIOLOGICAL ABSTRACTS, vol. 93, no. 8, 15 April 1992 Philadelphia, PA, US; abstract no. 89531, M. O. FAVOROV ET AL. 'Anti-HCV of IgM and IgG classes. ' page 606; & VOPR. VIRUSOL., vol. 36, no. 4, 1991 pages 281-284,

## Description

### FIELD OF THE INVENTION

This invention relates generally to interferon therapy, and more particularly, relates to the monitoring of anti-HCV IgM and anti-HCV IgG in an individual's test sample and determining a ratio as an indication of the clinical effectiveness and/or clinical outcome of interferon therapy used to treat an individual infected with chronic HCV.

### BACKGROUND OF THE INVENTION

Greater than 90% of cases of transfusion hepatitis worldwide are attributed to non-A, non-B hepatitis (NANBH). The predominant etiological agent of NANBH, termed Hepatitis C virus (HCV), has been cloned. An immunodominant region designated as c-100, encoded by the putative nonstructural (NS)-4 genomic region, has been expressed, purified, and incorporated into immunoassays which are useful in the detection of antibody to HCV in infected test samples. See, for example, Q.-L. Choo et al., Science 244:359-362 (1989); H. J. Alter et al., N. Engl. J. Med. 321:1494-1500(1989); J. I. Esteban et al., Lancet ii:294-297 (1989); G. Huo et al., Science 244:362-364 (1989); T. Miyamura et al., Proc. Natl. Acad. Sci. USA 87:983-987 (1990); and C. L. Van der Poel et al., Lancet ii:297-298 (1989).

It is known that a percentage of individuals who develop NANBH (HCV) infections progress to the chronic state of the disease, where liver function is impaired and death ultimately may result in severe cases. Interferon therapy has been shown to improve liver function in a proportion of patients with NANB hepatitis. From about thirty percent (30%) to about seventy percent (70%) of chronic Hepatitis C cases respond to alpha interferon (IFN) therapy. Although certain clinical features are associated with a likelihood of non-response to therapy such as age, duration of disease and existence of cirrhosis, no HCV-specific biochemical markers have been identified to predict response to therapy. Interferon induced normalization of serum alanine aminotransferase (ALT) levels is, to date, the only endpoint indicating response to IFN therapy. Biochemical relapse is predicted by increasing serum ALT levels. ALT levels, however, are not considered to be an HCV-specific marker molecule. Furthermore, ALT elevations may not occur for several months after viremia recurrence.

In addition to ALT elevation, recurrence of HCV viremia as measured by PCR amplification during or after interferon treatment is discussed as a possible predictor for relapse. However, PCR techniques are not practical for routine screening in a general clinical laboratory setting. Furthermore, the variance of PCR results from laboratory to laboratory is very high. Zaaijer et al., Lancet 341:722-24 (1993). Lastly, there is limited evidence that changes in anti-HCV IgG levels may change during IFN treatment.

It would be advantageous to provide a means for predicting the clinical outcome of interferon therapy in an individual receiving such therapy for NANB (HCV) infection. Such a means would be useful in that the response to therapy could be monitored, and the state of disease could be assessed to determine remission or relapse after therapy.

### SUMMARY OF THE INVENTION

The present invention provides a means for determining the effectiveness of interferon therapy for individuals receiving such therapy for HCV infections. Briefly, test samples from an individual are taken before the start of interferon therapy and assayed to determine the pre-interferon therapy amount of anti-HCV IgG and to determine the amount of anti-HCV IgM. A ratio of the pre-interferon therapy amount of anti-HCV IgG to the pre-interferon therapy amount of anti-HCV IgM is determined, wherein a statistical difference between the IgG/IgM ratio of non-responders and responders indicates the likelihood of the individual's response to interferon therapy. WO 94/04922 discloses an assay based upon a difference in IgM or IgG levels following interferon therapy.

### DETAILED DESCRIPTION OF THE INVENTION

The present invention provides a means for determining the effectiveness of interferon therapy for individuals receiving such therapy for HCV infections. The invention comprises assaying for the presence of anti-HCV IgG and anti-HCV IgM, determining a ratio of the pre-interferon therapy amount of anti-HCV IgG to the pre-interferon therapy amount of anti-HCV IgM, and using this ratio to determine clinical effectiveness of interferon therapy in an individual infected with HCV.

The present invention employs an immunoassay which utilizes specific binding members. A "specific binding member," as used herein, is a member of a specific binding pair. That is, two different molecules where one of the molecules through chemical or physical means specifically binds to the second molecule. Therefore, in addition to antigen and antibody specific binding pairs of common immunoassays, other specific binding pairs can include biotin and avidin, carbohydrates and lectins, complementary nucleotide sequences, effector and receptor molecules, cofactors and enzymes, enzyme inhibitors and enzymes, and the like. Furthermore, specific binding pairs can include members that are analogs of the original specific binding members, for example, an analyte-analog. Immunoreactive specific binding members include antigens, antigen fragments, antibodies and antibody fragments, both monoclonal and polyclonal, and complexes thereof, including those formed by recombinant DNA molecules. The term "hapten", as used herein, refers to a partial antigen or non-protein binding member which is capable of binding to an antibody, but which is not capable of eliciting antibody formation unless coupled to a carrier protein.

A "capture reagent", as used herein, refers to an unlabeled specific binding member which is specific either for the analyte as in a sandwich assay, for the indicator reagent or analyte as in a competitive assay, or for an ancillary specific binding member, which itself is specific for the analyte, as in an indirect assay. The capture reagent can be directly or indirectly bound to a solid phase material before the performance of the assay or during the performance of the assay, thereby enabling the separation of immobilized complexes from the test sample.

Test samples which can be tested by the methods of the present invention described herein include human and animal body fluids such as whole blood, serum, plasma, cerebrospinal fluid, urine, biological fluids such as cell culture supematants, tissue specimens and cell specimens.

An enhancer can be used to detect the generated signal in the assay. By "enhancer" is meant a moiety which can bolster a signal generated in an immunoassay, thereby amplifying the generated signal. Several methods of enhancing and amplifying a signal generated in an immunoassay are known in the art. Also, the use of a signal enhancer such as the use of avidin-biotin also is known. For example, U. S. Patent No. 4,228,237 to Hevey et al. describes the use of a biotin labelled specific binding substance for a ligand used in a method which also employs an enzyme labelled with avidin. The use of a biotin-anti-biotin system is described in European Patent Application No. 160,900, published on November 13, 1985.

The term "probe," as used herein, means a member of the specific binding pair attached to an "enhancer" compound. An "enhancer" compound can be any compound used in the assay which can enhance the signal generated by the signal generating compound. Thus, enhancer compounds include haptens such as biotin, and also include fluorescein, di-nitrophenol, and the like.

The indicator reagent comprises a signal generating compound (label) which is capable of generating a measurable signal detectable by external means conjugated (attached) to a specific binding member. "Specific binding member," as used herein, means a member of a specific binding pair. That is, two different molecules where one of the molecules through chemical or physical means specifically binds to the second molecule. In addition to being an antibody member of a specific binding pair for HCV, the indicator reagent also can be a member of any specific binding pair, including either hapten-anti-hapten systems such as biotin or anti-biotin, avidin or biotin, a carbohydrate or a lectin, a complementary nucleotide sequence, an effector or a receptor molecule, an enzyme cofactor and an enzyme, an enzyme inhibitor or an enzyme, and the like. An immunoreactive specific binding member can be an antibody, an antigen, or an antibody/antigen complex that is capable of binding either to HCV as in a sandwich assay, to the capture reagent as in a competitive assay, or to the ancillary specific binding member as in an indirect assay. Thus, if an enhancer is utilized in the assay, the indicator reagent comprises a signal generating compound conjugated to an enhancer-specific compound (enhancer compound binding member), such as biotin or anti-biotin, avidin or biotin, and others known to those skilled in the art. For example, if the enhancer compound utilized is biotin, then anti-biotin, or avidin, can be used as the enhancer-specific compound.

The various signal generating compounds (labels) contemplated include a chromogen such as bromo-chloro-indole-phosphate (BCIP), catalysts such as enzymes, luminescent compounds such as fluorescein and rhodamine, chemiluminescent compounds such as acridinium, phenanthridinium or 1,2-dioxetane compounds, radioactive elements, and direct visual labels. Examples of enzymes include alkaline phosphatase, horseradish peroxidase, beta-galactosidase, and the like. The selection of a particular label is not critical, but it will be capable of producing a signal either by itself or in conjunction with one or more additional substances, such as the use of enzyme substrates when enzymes are employed as the signal generating compound.

It is contemplated that the reagent employed for the assay can be provided in the form of a kit with one or more containers such as vials or bottles, with each container containing a separate reagent such as a monoclonal antibody, or a cocktail of monoclonal antibodies, employed in the assay.

The assay configuration may involve the use of a solid phase in performance of the present invention. A "solid phase", as used herein, refers to any material which is insoluble, or can be made insoluble by a subsequent reaction. The solid phase can be chosen for its intrinsic ability to attract and immobilize the capture reagent. Alternatively, the solid phase can retain an additional receptor which has the ability to attract and immobilize the capture reagent. The additional receptor can include a charged substance that is oppositely charged with respect to the capture reagent itself or to a charged substance conjugated to the capture reagent. As yet another alternative, the receptor molecule can be any specific binding member which is immobilized upon the solid phase and which has the ability to immobilize the capture reagent through a specific binding reaction. The receptor molecule enables the indirect binding of the capture reagent to a solid phase material before the performance of the assay or during the performance of the assay. If an assay device is utilized to perform the assays of the present invention, it can have many configurations, several of which are dependent upon the material chosen as the solid phase. For example, the solid phase can include any suitable porous material. By "porous" is meant that the material is one through which the test sample can easily pass and includes both bibulous and non-bibulous solid phase materials. In the present invention, the solid phase can include a fiberglass, cellulose, or nylon pad for use in a pour and flow-through assay device having one or more layers containing one or more of the assay reagents; a dipstick for a dip and read assay; a test strip for wicking (e.g., paper) or thin layer chromatographic or capillary action (e.g., nitrocellulose) techniques; or other porous or open pore materials well known to those skilled in the art (e.g., polyethylene sheet material). The solid phase, however, is not limited to porous materials. The solid phase can also comprise polymeric or glass beads, microparticles, tubes, sheets, plates, slides, wells, tapes, test tubes, or the like, or any other material which has an intrinsic charge or which can retain a charged substance.

Natural, synthetic, or naturally occurring materials that are synthetically modified, can be used as a solid phase including polysaccharides, e.g., cellulose materials such as paper and cellulose derivatives such as cellulose acetate and nitrocellulose; silica; inorganic materials such as deactivated alumina, diatomaceous earth, MgSO₄, or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon); porous gels such as silica gel, agarose, dextran, and gelatin; polymeric films such as polyacrilamide; and the like. The solid phase should have reasonable strength or strength can be provided by means of a support, and it should not interfere with the production of a detectable signal.

Preferred solid phase materials for flow-through assay devices include filter paper such as a porous fiberglass material or other fiber matrix materials. The thickness of such material is not critical, and will be a matter of choice, largely based upon the properties of the sample or analyte being assayed, such as the fluidity of the test sample.

To change or enhance the intrinsic charge of the solid phase, a charged substance can be coated directly to the material or onto microparticles which are then retained by a solid phase support material. Alternatively, microparticles can serve as the solid phase, by being retained in a column or being suspended in the mixture of soluble reagents and test sample, or the particles themselves can be retained and immobilized by a solid phase support material. By "retained and immobilized" is meant that the particles on or in the support material are not capable of substantial movement to positions elsewhere within the support material. The particles can be selected by one skilled in the art from any suitable type of particulate material and include those composed of polystyrene, polymethylacrylate, polypropylene, latex, polytetrafluoroethylene, polyacrylonitrile, polycarbonate, or similar materials. The size of the particles is not critical, although it is preferred that the average diameter of the particles be smaller than the average pore size of the support material being used.

Solid supports are known to those in the art and include the walls of wells of a reaction tray, test tubes, polystyrene beads, magnetic beads, nitrocellulose strips, membranes, microparticles such as latex particles, chips of glass, plastic, derivatized plastic, metal and silicon, and others.

Accordingly, a test sample which may contain anti-HCV IgG and anti-HCV IgM is tested for anti-HCV IgG by contacting the sample with a solid support to which HCV antigen has been attached, to form a mixture. This mixture is incubated for a time and under conditions sufficient to form HCV antigen/antibody complexes. Then, a probe comprising a mammalian anti-human IgG to which an enhancer has been attached is contacted with the HCV antigen/antibody complexes, to form a second mixture. This second mixture is incubated for a time and under conditions sufficient to form a second mixture reaction product. Next, an indicator reagent which comprises an enhancer compound binding member and a signal generating compound capable of generating a measurable signal is contacted with the second mixture reaction product . This third mixture is incubated for a time and under conditions sufficient to form indicator reagent reaction products. The presence and/or amount of HCV IgG is determined by detecting the signal generated. The amount of HCV IgG present in the test sample is proportional to the signal generated.

Another assay comprises an assay wherein a test sample which may contain anti-HCV IgG is contacted with a solid support to which HCV antigen has been attached, to form a mixture. This mixture is incubated for a time and under conditions sufficient to form HCV antigen/antibody complexes. Then, an indicator reagent which comprises a signal generating compound capable of generating a measurable signal attached to a specific binding member for anti-HCV IgG is contacted with the complexes, to form a second mixture. This second mixture is incubated for a time and under conditions sufficient to form a reaction. The presence and/or amount of anti-HCV IgG present in the test sample is determined by detecting the signal generated. The amount of anti-HCV IgG present in the test sample is proportional to the signal generated.

In yet another assay configuration, a test sample is contacted with mammalian anti-human IgG which is coated on a solid phase, and reacted for a time and under conditions sufficient for human IgG/anti-human IgG complexes to form. These complexes then are contacted with a probe which comprises at least one HCV antigen selected from HCV core, NS3 (viral protease) and NS4 (unknown function) attached to an enhancer compound. The preferred enhancer compound is biotin. These are reacted for a time and under conditions sufficient to form antigen/antibody/antibody complexes. Next, these complexes are contacted with an indicator reagent comprising a signal generating compound conjugated to an enhancer compound binding member. The most preferred signal generating compound is the enzyme alkaline phosphatase. The most preferred enhancer compound binding member is anti-biotin. The resultant mixture is reacted for a time and under conditions sufficient for a reaction to occur. If an enzyme is utilized, the signal is detected and measured after addition of an enzyme substrate. The amount of HCV IgG present in the test sample is proportional to the signal generated.

In a likewise fashion, a test sample which may contain anti-HCV IgG and anti-HCV IgM is tested for anti-HCV IgM by contacting the sample with a solid support to which HCV antigen has been attached, to form a mixture. This mixture is incubated for a time and under conditions sufficient to form HCV antigen/antibody complexes. Then, a probe comprising a mammalian anti-human IgM to which an enhancer has been attached is contacted with the HCV antigen/antibody complexes, to form a second mixture. This second mixture is incubated for a time and under conditions sufficient to form a second mixture reaction product Next, an indicator reagent which comprises an enhancer compound binding member and a signal generating compound capable of generating a measurable signal is contacted with the second mixture reaction product . This third mixture is incubated for a time and under conditions sufficient to form indicator reagent reaction products. The presence and/or amount of HCV IgM is determined by detecting the signal generated. The amount of HCV IgM present in the test sample is proportional to the signal generated.

Another assay comprises an assay wherein a test sample which may contain HCV IgM is contacted with a solid support to which HCV antigen has been attached, to form a mixture. This mixture is incubated for a time and under conditions sufficient to form HCV antigen/antibody complexes. Then, an indicator reagent which comprises a signal generating compound capable of generating a measurable signal attached to a specific binding member for HCV IgM is contacted with the complexes, to form a second mixture. This second mixture is incubated for a time and under conditions sufficient to form a reaction. The presence and/or amount of HCV IgM present in the test sample is determined by detecting the signal generated. The amount of HCV IgM present in the test sample is proportional to the signal generated.

In yet another assay configuration, a test sample is contacted with mammalian anti-human IgM which is coated on a solid phase, and reacted for a time and under conditions sufficient for human IgM/anti-human IgM complexes to form. These complexes then are contacted with a probe which comprises at least one HCV antigen selected from HCV core, NS3 (viral protease) and NS4 (unknown function) attached to an enhancer compound. The preferred enhancer compound is biotin. These are reacted for a time and under conditions sufficient to form antigen/antibody/antibody complexes. Next, these complexes are contacted with an indicator reagent comprising a signal generating compound conjugated to an enhancer compound binding member. The most preferred signal generating compound is the enzyme alkaline phosphatase. The most preferred enhancer compound binding member is anti-biotin. The resultant mixture is reacted for a time and under conditions sufficient for a reaction to occur. If an enzyme is utilized, the signal is detected and measured after addition of an enzyme substrate. The amount of HCV IgM present in the test sample is proportional to the signal generated.

Yet other assay configurations can be adapted to detect anti-HCV IgG and anti-HCV IgM by practicing the teachings of this invention, and are contemplated to be within the scope of this invention. It is further contemplated that a test sample may be tested simultaneously for anti-HCV IgG and anti-HCV IgM, wherein anti-HCV IgG and anti-HCV IgM are labeled in such a way that the two classes of antibodies are distinguishable. For example, a test sample is contacted with a solid support to which HCV antigen has been attached, to form a mixture. This mixture is incubated for a time and under conditions sufficient to form HCV antigen/antibody complexes. Then, indicator reagents which comprise a signal generating compound capable of generating a measurable signal attached to a specific binding member is contacted with the complexes. The specific binding member anti-human IgG is attached to a fluorescent label that fluoresces at one wavelength, whereas the specific binding member anti-human IgM is attached to a fluorescent label that fluoresces at a second wavelength. Both wavelengths can be read in one mixture.

When assaying for anti-HCV IgM, it is preferred that the test sample be treated such as to remove rheumatoid factor-like substances which may be present in the test sample and which may interfere with the performance of the assay. Such treatment can be performed in a variety of ways known to those skilled in the art and include preadsorbing the test sample with protein A or protein G, heat aggregated IgG, and the subjection of the test sample to an amount of anti-human IgG sufficient to bind a substantial amount of the interfering rheumatoid factor-like substances. The most preferred method for treating the test sample comprises diluting the test sample in a diluent sample buffer which contains an amount of goat anti-human IgG sufficient to bind the rheumatoid factor-like substances which may be present in the test sample. This dilution step preferably is performed prior to contacting the test sample with the capture reagent, HCV antigen. The preferred buffer is one which can remove any interfering IgG which may be present in the test sample. Thus, buffers which contain a sufficient quantity of anti-IgG can be used as the diluent sample buffer. Examples of buffers that can be used in the assay include Tris buffered saline, phosphate buffered saline, and others known to those skilled in the art. The most preferred buffer comprises a Tris buffered saline (pH 7.2) to which goat anti-human IgG has been added. Further, other compounds may be added to this buffer to block non-specific binding. The selection of these compounds depends upon the constituents chosen for the assay, and are within the ordinary skill of the artisan.

The origin of the mammalian anti-human IgG or mammalian anti-human IgM may be goat, rabbit, sheep, or other mammalian anti-human IgG or anti-human IgM known in the art Preferably, the mammalian origin of the anti-human IgG and anti-human IgM is goat

When HCV antigen is used as a capture reagent in the assays described herein, at least one HCV antigen is used, either when attached to a solid phase or in solution. These include HCV antigens from the core, NS3 and NS4 genomic regions. We have determined that HCV core is the most preferred antigen to utilize in performing the assays for detection of anti-HCV IgG and anti-HCV IgM antibodies, but that NS3 and NS4 also can be used alone or in any combination. Thus, HCV core antigen can be combined with NS3 and/or NS4, or other HCV antigens, and used as capture antigens in methods described herein.

It also is contemplated that a sandwich assay can be performed wherein a soluble capture reagent can include an analyte-specific binding member which has been bound to a charged substance such as an anionic substance. The present invention also can be used to conduct a competitive assay. In a competitive configuration, the soluble capture reagent again includes a specific binding member which has been attached to a charged substance, such as an anionic polymer, with which to bind a specific binding partner.

Alternatively, it also is contemplated that the assay can be performed by scanning probe microscopy, in which an analyte, analyte analog or analyte specific substance which has been bound to a test piece, is contacted with the test sample suspected of containing the analyte, incubated for a time and under conditions sufficient for a reaction to occur, and then the presence of analyte is determined by using scanning probe microscopy.

In accordance with the invention, standard curves are generated to determine the amount of anti-HCV IgG and anti-HCV IgM in a test sample from an HCV-infected patient. A "standard curve," as used herein, refers to a relationship established between the intensity of the signal generated by the antigen-antibody complexes formed in accordance with the assay methods of the invention and the amount of anti-HCV IgG or anti-HCV IgM in a sample.

The present invention will now be described by way of Examples, which are intended to demonstrate, but not to limit, the spirit and scope of the invention.

### SPECIMENS

A large cohort (N=182) of HCV-RNA positive patients with histologically proven chronic hepatitis were tested for Anti-HCV core and anti-HCV IgM in the assays described below. These patients participated in a BENELUX randomized trial of alpha-interferon (IF). Patients were classified as non-responders (NR), sustained-responders (SR) or transient-responders (TR) based on their level of the liver enzyme ALT. Of the 182 samples, 98 samples were classified as non-responders, 38 were classified as sustained-responders and 46 were classified as transient-responders.

Serum samples were collected at three different time points: 1) prior to the start of IFN therapy, 2) at the end of IFN therapy, and 3) at a follow-up period.

### EXAMPLES

### Example 1

### Anti-HCV Core IgG Assay

Calibrators were tested at dilutions of 1:1 (neat), 1:2, 1:4, 1:8, 1:16, 1:32 and 1:64. Test samples were tested neat and at dilutions of 1:31 and 1:961. All samples were diluted with a predilution buffer. Such a buffer comprising a buffered salt solution is well understood in the art and chosen from among those buffers known not to interfere with other reagents in the assay methods described herein.

The test samples and calibrators were further diluted in the assay at a dilution factor of 1:81 with specimen diluent which comprised TRIS buffer, 0.5% Triton X-100®, 0.5% Tween 20, E. coli lysate/CKS, and bovine and goat sera, commercially available from Abbott Laboratories, Abbott Park, IL. Two hundred ul of each diluted sample were incubated with a polystyrene bead coated with HCV core antigen at 40°C +/- 2°C for 60 minutes +/- 5 minutes in a Commander® Dynamic Incubator, commercially available from Abbott Laboratories, in the static mode. After incubation, unbound materials were aspirated and the beads were washed with water. Two hundred ul of diluted conjugate comprising goat anti-human IgG conjugated to the enzyme horseradish peroxidase were added to each well and the beads were incubated at 40°C +/- 2°C for 30 minutes +/- 5 minutes in a Commander® Incubator in the static mode. Unbound materials were aspirated and the beads were washed. Color development was achieved by adding o-Phenylenediamine (OPD) solution containing hydrogen peroxide to the beads, and, after incubation for approximately 30 minutes at room temperature, a yellow-orange color developed in proportion to the amount of anti-HCV which was bound to the bead. The enzyme reaction was stopped by adding 1 ml of 1N H₂SO₄. The intensity of the color was measured using a spectrophotometer at a wavelength of 492 nm.

The anti-HCV absorbance values were translated into IgG units by doing a "point to point" linear regression of the calibrator curve. Briefly, each dilution of the calibration curve was assigned a "concentration related" value as follows: neat, 64 units; 2-fold, 32 units; 4-fold, 16 units; 8-fold, 8 units; 16-fold, 4 units; 32-fold, 2 units; and 64-fold, 1 unit.

The cutoff value for the assay was calculated by determining the negative control mean absorbance value (NCx) and the positive control mean absorbance value (PCx) in the following formula: Cutoff value = NCx + (0.25)PCx. A concentration related value for the cutoff value was determined by linear regression for the line derived from the 8-fold and 16-fold dilutions.

A "concentration value" of 1.0 was assigned to the concentration related value calculated above for the cutoff point. A concentration value then was assigned to each dilution of the calibration curve by dividing its designated concentration related value (i.e., 64 units, 32 units . . . 1 unit) by the concentration related value determined for the cutoff value. Next, a concentration value was calculated for each sample tested by 1) determining between which two dilutions of the calibration curve the O.D. value of the test sample falls; and 2) performing a linear regression for the line derived from the two dilution points that bracket the absorbance value of the unknown test sample. The calculated concentration value was multiplied by the dilution factor of the sample tested to arrive at IgG units of the neat test sample.

### Example 2

### Automated Anti-HCV IgG Assay

Anti-HCV core IgG can be measured in an automated immunoassay utilizing an automated instrument for performing microparticle enzyme immunoassays (MEIA), the Abbott IMx® analyser, commercially available from Abbott Laboratories. In the assay method, polystryrene microparticles coated with recombinant HCV core protein are reacted with a test sample, washed and the presence of anti-HCV IgG is detected by the use of an anti-human IgG alkaline-phosphatase antibody conjugate. MUP is added as the substrate and the resulting signal is recorded by the front surface fluorometer of the IMx® analyser.

One of the advantages of this assay format is that the test sample is tested at a final dilution of 1:2000 which is performed by the IMx® analyser with no pretreatment required. A standard curve is generated using serial dilutions of a HCV positive reference plasma IMx measuring rates are converted by log/logit transformation or a four-parameter logistic curve fitting method and expressed as arbitrary anti-HCV IgG units.

### Example 3

### Anti-HCV Core IgM Assay

The test samples, calibrators and controls were diluted at a dilution factor of 1:231 (the so-called "231" dilution) wherein the sample first was diluted by using 10 ul of the sample and 200 ul of sample diluent, mixed and then diluted again using 20 ul of the mixture and an additional 200 ul of sample diluent.

The diluted samples were incubated with polystyrene beads coated with HCV core antigen at 40°C +/- 2°C for 150 minutes +/- 10 minutes in a Commander® Dynamic Incubator in the rotation mode. After incubation, unbound materials were aspirated and the beads were washed with water. Two hundred ul of diluted conjugate comprising goat anti-human IgM conjugated to the enzyme horseradish peroxidase were added to each well and the beads were incubated at 40°C +/- 2°C for 60 minutes +/- 5 minutes in a Commander® Dynamic Incubator in the rotation mode. Unbound materials were aspirated and the beads were washed. Color development was achieved by adding OPD solution containing hydrogen peroxide to the beads, and, after incubation for approximately 30 minutes at room temperature, a yellow-orange color developed in proportion to the amount of anti-HCV IgM which was bound to the bead. The enzyme reaction was stopped by adding 1 ml of 1N H₂SO₄. The intensity of the color was measured using a spectrophotometer at a wavelength of 492 nm.

Three calibrators with three different amounts of anti-HCV IgM were tested in the assay to generate a standard curve. The calibrators comprised an anti-HCV IgM positive serum tested at the dilutions of 1:10 and 1:20 along with HCV negative serum. A scale of 100 units was defined as the quantitation range. Thus, a linear regression of the three calibrators provided a method for HCV IgM quantitation.

### Example 4

### IgG/IgM Ratio Analysis

The IgG units were calculated for each test sample as described in Example 1 above. The IgM units for each test sample also were calculated as described in Example 3 above. Tables 1 and 2 below set forth the mean IgG units and IgM units values, respectively, for pre-interferon therapy samples.

**Table 1**

| IgG Units | N | Mean | Std. Dev. |
|---|---|---|---|
| Non-Responders | 98 | 469 | 668 |
| Transient-Responders | 46 | 723 | 1372 |
| Sustained-Responders | 38 | 1045 | 1239 |

**Table 2**

| IgM Units | N | Mean | Std. Dev. |
|---|---|---|---|
| Non-Responders | 102 | 121 | 233 |
| Transient-Responders | 48 | 44 | 85 |
| Sustained-Responders | 39 | 28 | 65 |

An IgG/IgM ratio was calculated for each sample tested by dividing the IgG units of a sample by the IgM units for that sample. The mean IgG/IgM ratios for the pre-interferon therapy sample for each group of patients are presented in Table 3.

**Table 3**

| IgG/IgM Ratio | N | Mean | Std. Dev. |
|---|---|---|---|
| Non-Responders | 98 | 51 | 200 |
| Transient-Responders | 46 | 345 | 1900 |
| Sustained-Responders | 38 | 109 | 156 |

The IgG/IgM ratios of the pre-interferon samples were statistically compared using the Mann-Whitney U-test. Altman, Douglas, G., Practical Statistics for Medical Research (Chapman & Hall: London, 1991). The statistical comparison is set forth in Table 4, wherein the data is presented as p-values.

**Table 4**

| Patient Groups | IgG/IgM Ratio Significance |
|---|---|
| Non-Responders vs. Sustained Responders | <0.0001 |
| Non-Responders vs. Transient-Responders | 0.0059 |
| Sustained-Responders vs. Transient Responders | NS¹ |

| | |
|---|---|
| ¹NS=not significant | |

Thus, the results show that there is a statistical difference between non-responders and responders based on the pre-interferon therapy ratio of IgG/IgM.

## Claims

1. A method for determining the effectiveness of interferon therapy in an individual infected with HCV, comprising:
(a) determining the pre-interferon therapy amount of anti-HCV IgG in a test sample of a patient infected with HCV;
(b) determining the pre-interferon therapy amount of anti-HCV IgM in a test sample of a patient infected with HCV;
(c) determining a ratio of the pre-interferon therapy amount of anti-HCV IgG to the pre-interferon therapy amount of anti-HCV IgM,
wherein a statistical difference between the IgG/IgM ratio of non-responders and responders indicates the likelihood of said individual's response to interferon therapy.

## Patentansprüche

1. Verfahren zur Bestimmung der Wirksamkeit der Interferontherapie bei Individuen, die mit HCV infiziert sind, das folgendes umfaßt:
(a) Bestimmen der vor-Interferontherapie-Menge von anti-HCV IgG in einer Testprobe eines mit HCV infizierten Patienten;
(b) Bestimmen der vor-Interferontherapie-Menge von anti-HCV IgM in einer Testprobe eines mit HCV infizierten Patienten;
(c) Bestimmen eines Verhältnisses der vor-Interferontherapie-Menge von anti-HCV IgG zu der vor-Interferontherapie-Menge von anti-HCV IgM,
wobei ein statistischer Unterschied zwischen dem IgG/IgM Verhältnis von nicht ansprechenden Patienten und ansprechenden Patienten die Wahrscheinlichkeit des Ansprechens des Individuums auf die Interferontherapie anzeigt.

## Revendications

1. Procédé de détermination de l'efficacité d'une thérapie par interféron chez un individu infecté par le HCV, comprenant
(a) la détermination de la quantité d'IgG anti-HCV, avant la thérapie par interféron, dans un échantillon d'analyse d'un patient infecté par le HCV ;
(b) la détermination de la quantité d'IgM anti-HCV, avant la thérapie par interféron, dans un échantillon d'analyse d'un patient infecté par le HCV ;
(c) la détermination du rapport de la quantité d'IgG anti-HCV avant la thérapie par interféron à la quantité d'IgM anti-HCV avant la thérapie par interféron,
une différence statistique entre le rapport IgG/IgM des non répondants et celui des répondants indiquant la probabilité de la réponse dudit individu à la thérapie par interféron.
